# EUROPEAN PATENT APPLICATION

(11) **EP 3 170 512 A1**
(43) Date of publication of application: **24.05.2017**
(21) Application number: 15818176.8
(22) Date of filing: 06.07.2015
(51) Int. Cl.: A61K 45/06, A61K 9/14, A61K 31/4184, A61K 31/4439, A61K 31/47, A61K 38/00, A61K 47/34, A61P 9/10, A61P 43/00

(54) **PHARMACEUTICAL COMPOSITION**

(30) Priority: 07.07.2014 JP 2014139995
(71) Applicant: Sentan Pharma Inc., Fukuoka-shi, Fukuoka 8120027 (JP)
(72) Inventor: EGASHIRA Kensuke, Fukuoka-shi Fukuoka 812-0027 (JP)
(74) Representative: V.O.
(86) International application number: PCT/JP2015/069425
(87) International publication number: WO 2016/006577

(57) **Abstract**

A pharmaceutical composition for use in treatment or prevention of disorders caused by ischemia contains a mitochondrial damage inhibitor, an anti-inflammatory agent, and a biocompatible particle that encloses both or each of the above mitochondrial damage inhibitor and the above anti-inflammatory agent. The above-mentioned biocompatible particle may be a poly(lactic-co-glycolic acid) copolymer having a number mean particle size of 2.5 to 1000 nm or a polyethylene glycol modification thereof.

## Description

### Technical Field

The present disclosure relates to pharmaceutical compositions.

### Background Art

Ischemic diseases such as acute myocardial infarction and cerebral infarction are the most serious diseases that threaten vital prognosis of patients. According to the World Health Organization, the ischemic disease is the number one cause of death in human. The most important factor that determines the prognosis for the patient with the ischemic disease is an infarct size. Because of this, the only optimum treatment that is currently carried out is an early reperfusion therapy that early resumes the blood flow and gets rid of the ischemia, thereby reducing the infarct size. But, the recovery of the blood flow by reperfusion *per se* promotes irreversible necrosis of cardiac muscle cells; and what has been known is reperfusion injury which decreases effects of the reperfusion therapy to reduce the infarct. In an animal experiment, the reperfusion injury wipes out the effect of the reperfusion therapy to reduce the infarct size by about 50%.

Due to the reperfusion injury, a therapeutic effect of reperfusion therapy, that is, the effect of reducing the infarct size is insufficient. Because of this, the long term prognosis for the patient with the ischemic disease has not improved at all. For example, the large scale registry of early reperfusion therapy in the United States reports that improvement in the reduction of the infarct size and improvement in the prognosis for the patient are sufficient (see Non Patent Literature 1).

Many factors are involved in disease conditions of the ischemia-reperfusion injury (see Non Patent Literature 2). Of those, major factors are (1) mitochondrial damages (in particular, damages or failure ascribed to opening of mitochondrial permeability-transition pore, hereinafter referred to simply as "mPTP") in an early phase following ischemia (within 10 minutes) and (2) inflammation (in particular, infiltration of activated monocytes) in a late phase (one to three hours) following ischemia.

Having said that, most of the conventional basic research have been conducted using protocols for carrying out therapeutic intervention with either one of the above (1) and (2) or another factor that is different from (1) and (2) as a target. This is because what has been commonly accepted is that as long as one important factor in mechanisms of the ischemia-reperfusion injury is sufficiently intervened, additional therapeutic effects are brought about by interaction; and intervention in plural factors is hard to produce additional therapeutic effects (see Non Patent Literature 2, in particular, FIG. 3). Thus far, in order to apply results of the basic research to practical use, a lot of clinical studies have been conducted; but many of them are unable to reach endpoints and the results of the clinical studies have been unfavorable (see Non Patent Literature 3).

Several reasons are thought to be accountable for the unfavorable result of the clinical studies. Examples of the reasons include the fact that the effect of inhibiting the ischemia-reperfusion injury (about 50% reduction in terms of the infarct size), the effect being achieved in an animal experiment, was not sufficient.

Non Patent Literatures 4 to 7 have reported that combined use of two agents at small amount (subthreshold dose) produces more effects than use of either one of the agents, wherein the agent, when used solely, hardly produces effects at such a subthreshold dose. Even in the case where two agents were used in combination at an effective dose, the obtained effect was not equal to or did not exceed the sum of effects obtained when each of the agents was used solely.

In addition, in all of the above Non Patent Literatures 4 to 7, the drug is administered prior to ischemia and is not administered at the time of reperfusion. With an eye to clinical applications, it is necessary for the drug to be administered when a patient receives reperfusion therapy instead of administration of the drug prior to the development of ischemia.

### Citation List

### Non Patent Literature

Non Patent Literature 1: Daniel S et al., Door-to-Balloon Time and Mortality among Patients Undergoing Primary PCI., N. Engl. J. Med., 2013, 369, 901-909
Non Patent Literature 2: Prasad A et al., Reperfusion injury, microvascular dysfunction, and cardioprotection: the "dark side" of reperfusion., Circulation, 2009, 120(21), 2105-2112
Non Patent Literature 3: Kloner RA et al., Current state of clinical translation of cardioprotective agents for acute myocardial infarction., Circ. Res., 2013, 113(4), 451-463
Non Patent Literature 4: Mao X et al., N-acetylcysteine and allopurinol confer synergy in attenuating myocardial ischemia injury via restoring HIF-1α/HO-1 signaling in diabetic rats., PLoS One, 2013, 8(7), e68949
Non Patent Literature 5: Manickavasagam S et al., The cardioprotective effect of a statin and cilostazol combination: relationship to Akt and endothelial nitricoxide synthase activation. Cardiovasc. Drugs Ther., 2007, 21(5), 321-330
Non Patent Literature 6: Aitchison KA et al., Potential interactions between iloprost and SIN-1 on platelet aggregation and myocardial infarct size in vivo., Eur. J. Pharmacol., 1999, 374(1), 59-69
Non Patent Literature 7: Ma J et al., Synergistic effects of caspase inhibitors and MK-801 in brain injury after transient focal cerebral ischaemia in mice., Br. J. Pharmacol., 1998, 124(4), 756-762

### Summary of Invention

### Technical Problem

As mentioned above, no effective treatment methods are available to prevent or reduce the ischemia-reperfusion injury and sufficiently reduce the infarct size. To improve the quality of life and prognosis for the patient with the ischemic disease such as acute myocardial infarction and cerebral infarction, development of an innovative treatment method for the ischemia-reperfusion injury is an imminent issue.

The present disclosure has been made in light of the above current circumstances; and an object thereof is to provide pharmaceutical compositions capable of reducing the infarct size as small as possible.

### Solution to Problem

Although the commonly accepted theory is, as described above, that the intervention in plural factors involved in the mechanism of the ischemia-reperfusion injury is hard to produce additional therapeutic effects, there is no need, for the purpose of achieving a maximum effect of reducing the infarct size, to stick to the intervention in one factor that has been found to be important with respect to molecular cell biology from the basic research. Meanwhile, blood flow is insufficient in an ischemia reperfusion site, which is attributed to microvascular damage such as endothelial cell injury or blood clots and inflammation. Because of this, there is a problem that an effective amount of drug is not delivered to the ischemia reperfusion site even if the drug is intravenously or intracoronarily administered at the time of reperfusion. Thus, even if a drug that intervenes in the above (1) and (2) is administered in the form of bulk at the time of reperfusion, an effective amount of the drug is not delivered to the reperfusion site and it is therefore thought that the effect of reducing the infarct size is insufficient.

The present inventor has discovered that the inhibitory effect on the ischemia-reperfusion injury, that is, the effect of reducing the infarct size is able to be maximized by applying a drug delivery system (DDS) to intervene the above (1) and (2) of the ischemia-reperfusion injury at the same time.

Accordingly, a pharmaceutical composition for use in treatment or prevention of a disorder caused by ischemia according to the aspect of the present disclosure comprises:
a mitochondrial damage inhibitor;
an anti-inflammatory agent; and
a biocompatible particle that encloses both or each of the above mitochondrial damage inhibitor and the above anti-inflammatory agent.

In this case, the above biocompatible particle may comprise
a poly(lactic-co-glycolic acid) copolymer having a number mean particle size of 2.5 to 1000 nm or a polyethylene glycol modification thereof.

Further, the above disorder caused by ischemia may be
ischemia-reperfusion injury.

Further, the above mitochondrial damage inhibitor may be
cyclosporine or Mitochondrial division inhibitor 1.

Further, the above anti-inflammatory agent may be
selected from the group consisting of: pitavastatin, irbesartan, pioglitazone, and C-C chemokine receptor type 2 inhibitors.

Further, the above pharmaceutical composition may be administered to a patient in combination with reperfusion therapy.

Further, the above disorder caused by ischemia may be
a disorder in an organ that has come to be in an ischemic state.

### Advantageous Effects of Invention

According to the present disclosure, the infarct size is able to be reduced as small as possible.

### Brief Description of Drawings

FIG. 1 is a figure showing images of the heart specimen obtained from the model mouse of myocardial ischemia-reperfusion. The mouse was administered with normal saline, FITC, and FITC nanoparticle (FITC-NP) at the time of reperfusion;
FIG. 2 is a figure showing the fluorescence intensity acquired from image analysis of the heart specimen. The specimen was obtained from the model mouse of myocardial ischemia-reperfusion;
FIG. 3 is a figure showing a fluorescence image of the heart specimen from the group administered with FITC-NP;
FIG. 4 is a figure showing an optical image and a fluorescence image of the mitochondrial fraction. The fraction was extracted from the model mouse of myocardial ischemia-reperfusion;
FIG. 5 is a figure showing data obtained by quantifying FITC in the mitochondrial fraction;
FIG. 6A is a figure showing an image of cultured cardiac muscle cells from the heart specimen from the group administered with FITC-NP. The mitochondria are stained in cells; FIG. 6B is a figure showing an image of cultured cardiac muscle cells exposed to hydrogen peroxide to be affected by FITC nanoparticles; FIG. 6C is a figure showing an image obtained by overlapping the image shown in FIG. 6A on the image shown in FIG. 6B;
FIG. 7 is a figure showing FITC signals acquired by flow cytometry analysis of lymphocytes, neutrophils, and monocytes in the heart, the blood, and the spleen of the model mouse of myocardial ischemia-reperfusion six hours after reperfusion;
FIG. 8 is a figure showing the effect of combined use to reduce the area of infarct, wherein cyclosporine A nanoparticles (CsA-NP) were used in combination with pitavastatin nanoparticles (pitavastatin-NP) in the model mouse of myocardial ischemia-reperfusion;
FIG. 9 is a figure showing the effect of combined use to reduce the area of infarct, wherein CsA-NPs were used in combination with irbesartan nanoparticles (irbesartan-NP) in the model mouse of myocardial ischemia-reperfusion;
FIG. 10 is a figure showing the effect of combined use to reduce the area of infarct, wherein CsA-NPs were used in combination with pioglitazone nanoparticle (pioglitazone-NP) in the model mouse of myocardial ischemia-reperfusion;
FIG. 11 is a figure showing the effects of CsA-NP, pitavastatin-NP, irbesartan-NP, and pioglitazone-NP to reduce the area of infarct in the model mouse of myocardial ischemia-reperfusion, which mouse being devoid of cyclophilin D (CypD), and a ratio of the infarct region to the ischemic region in the model mouse of myocardial ischemia-reperfusion, which mouse being devoid of CypD and C-C chemokine receptor type 2 (referred to also as "CCR2"); and
FIG. 12 is a figure showing the effect of CCR2 inhibitor nanoparticles (CCR2 inhibitor-NP) to reduce the area of infarct, in the model mouse of myocardial ischemia-reperfusion.

### Description of Embodiments

The embodiments according to the present disclosure will be described.

### (Embodiment 1)

First of all, the embodiment 1 of the present disclosure will be described. The pharmaceutical composition according to the present embodiment contains a mitochondrial damage inhibitor, an anti-inflammatory agent, and a biocompatible particle that encloses both or each of the mitochondrial damage inhibitor and the anti-inflammatory agent.

The mitochondrial damage inhibitor is an mPTP opening inhibitor which inhibits mitochondrial damages, for example, by directly or indirectly inhibiting the opening of mPTP. mPTP is composed of cyclophilin D, VDAC (voltage dependent anion channel), and the like and is a pore structure located at contact sites between the inner mitochondrial membrane and the outer mitochondrial membrane. To be specific, the mitochondrial damage inhibitor is cyclosporine. Cyclosporine is a cyclic polypeptide, has an action of inhibiting calcineurin, and is known as an immunosuppressant. It is known that cyclosporine binds to cyclophilin D and then removes cyclophilin D out of the inner membrane, thereby inhibiting the opening of mPTP. Among cyclosporine, cyclosporine A which is the most abundant in nature may be used as the mitochondrial damage inhibitor. In addition, derivatives of cyclosporine such as N-methyl-valine-cyclosporine and N-methyl-4-isoleucine-cyclosporine (NIM811) may be used as the mitochondrial damage inhibitor.

Further, examples of the mitochondrial damage inhibitor can include 2-aminoethoxydiphenyl borate (2-APB), bongkrekic acid, sangfehrin A, and inhalational anesthetics such as isoflurane, in addition to cGMP-phosphodiesterase inhibitors which inhibit the opening of mPTP via cGMP-dependent protein kinases, cGMP analogs (cGMP activators or stimulants), and nitric oxide-generating agents.

Besides those described above, Mitochondrial division inhibitor 1 (Mdivi-1, 3-(2,4-dichloro-5-methoxyphenyl)-2,3-dihydro-2-thioxo-4(1H)-quinazolinone or 3-(2,4-dichloro-5-methoxyphenyl)-2-sulfanil-4(3H)-quinazolinone) is suitable for the mitochondrial damage inhibitor. Mdivi-1 selectively inhibits mitochondrial division DRP (dynamin-related GTPase) and further inhibits mitochondrial division dynamin (Dnm1). Mitochondrial fusion and division are involved in apoptosis of cells; and Mdivi-1 inhibits mitochondrial damages to thereby inhibit the apoptosis.

Those commercially available can be used as the mitochondrial damage inhibitor. Further, the mitochondrial damage inhibitor can be produced by a known synthesis method, for example, a synthesis method utilizing a chemical reaction or an enzymatic reaction (for example, see Unexamined Japanese Patent Application Kokai Publication No. 2005-325061 for cyclosporine).

To select a compound having an action of inhibiting the opening of mPTP as mitochondrial damage inhibitor, a known method, for example, a method in which evaluation is carried out by image analysis or flow cytometry with a fluorescent dye calcein may be used. Further, the mitochondrial damage inhibitor may be quantified by using a measurement kit that is commercially available. To be more specific, an example of such a measurement kit is MitoProbe (trademark) Transition Pore assay kit (manufactured by Molecular Probes) or the like.

The anti-inflammatory agent may be a steroidal anti-inflammatory agent or a non-steroidal anti-inflammatory agent; and examples are piroxicam, diclofenac, propionic acids (naproxen, flurbiprofen, fenoprofen, ketoprofen, and ibuprofen), fenamates (mefenamic acid), indomethacin, sulindac, apazone, pyrazolone (phenylbutazone), salicylate (Aspirin), COX-2 inhibitors (celecoxib and rofecoxib), and the like.

In particular, what is suitable as the anti-inflammatory agent is ones that have an action of inhibiting infiltration or recruitment of activated monocytes or an action of inhibiting the activation of monocytes. Such an anti-inflammatory agent is, for example, pitavastatin, irbesartan, pioglitazone, or the like.

Inhibitors of CCR2 which a receptor for Monocyte chemoattractant protein-1 (MCP-1) are suitable as the anti-inflammatory agent as well.

Those commercially available can be used as the above anti-inflammatory agent. Further, the anti-inflammatory agent can be produced by a known synthesis method, for example, a synthesis method utilizing a chemical reaction or an enzymatic reaction. Besides, anti-inflammatory agents prepared by bringing a compound, a nucleic acid, a peptide, or the like into action on cells and carrying out screening by quantifying expression, secretion, or the like of inflammatory mediators can be also used.

The anti-inflammatory agent may be losartan, valsartan, candesartan cilexetil, telmisartan, olmesartan medoxomill, or the like.

The biocompatible particle may enclose both of the mitochondrial damage inhibitor and the anti-inflammatory agent; or the biocompatible particle may individually enclose the mitochondrial damage inhibitor and the anti-inflammatory agent.

The biocompatible particle can be produced from biocompatible polymers. The biocompatible polymers vary in its average chain length, leading to difference in inherent viscosity and polymer characteristics. Polymers used in the present embodiment are preferably those that possess biocompatibility, being less stimulative and less toxic to organisms and that are biodegradable, being broken down after administered and metabolized. Examples of the biodegradable polymer include polymers that are produced in microorganisms such as polyhydroxybutyrate or polyhydroxyvalerate; and natural polymers such as collagen, cellulose acetate, bacterial cellulose, high amylose corn starch, starch, or chitosan.

The biocompatible particle obtained from the biocompatible polymer preferably releases the enclosing mitochondrial damage inhibitor and anti-inflammatory agent in a sustained, that is, controlled fashion. Because of this, those with a molecular weight of 5,000 to 200,000 or a molecular weight 15,000 to 25,000 are for example preferred as the biocompatible polymer.

The biocompatible particle can be produced from biocompatible polymers, for example, biocompatible polyesters. The biocompatible polyester is a polyester synthesized by polymerizing one or more kinds of monomers selected from, for example, D,L-lactide, D-lactide, L-lactide, D,L-lactic acid, D-lactic acid, L-lactic acid, glycolide, glycolic acid, ε-caprolactone, ε-caprolactone, ε-hydroxyhexanoic acid, γ-butyrolactone, γ-hydroxybutyric acid, δ-valerolactone, δ-hydroxyvaleric acid, hydroxybutyric acid, malic acid, and the like. The biocompatible polymer is preferably polylactic acid, polyglycolic acid, lactic acid·glycolic acid copolymers, or lactic acid·aspartic acid copolymers, and, in particular, poly(lactic-co-glycolic acid) copolymer (PLGA) or polyethylene glycol/chitosan modified-PLGA (PEG/CS-PLGA).

PLGA as a biocompatible polymer is a copolymer composed of lactic acid or lactide and glycolic acid or glycolide at a ratio of, for example, 1:99 to 99:1, and preferably 3:1. PLGA may be synthesized from freely-selected monomers by a common method; or commercially available PLGA may be used. Examples of the commercially available PLGA include PLGA7520 (lactic acid: glycolic acid = 75:25, weight average molecular weight 20,000, manufactured by Wako Pure Chemical Industries, Ltd.). PLGA whose content of lactic acid and glycolic acid is from 25% by weight to 65% by weight is amorphous and is preferred in that such a PLGA is soluble in organic solvents such as acetone.

In cases where the above biocompatible particle is produced from PLGA, the biocompatible particle may be produced so as to contain PLGA with a particle size of less than 1,000 nm, for example, 2.5 to 1,000 nm, preferably 5 to 800 nm, more preferably 25 to 600 nm, still more preferably about 50 to 500 nm, and most preferably 200 to 400 nm. The particle size can be measured by a sieving method, a precipitation method, a microscope method, a light scattering method, a laser diffraction/scattering method, an electrical resistance test, or the like. The particle size can be expressed in Stokes radius, equivalent circular diameter, or equivalent spherical diameter depending on a method for measurement. In addition, the particle size may be number mean particle size, volume mean particle size, area mean particle size, or the like, which represents a mean obtained by subjecting plural particles to the measurement. For example, the number mean particle size is a mean particle size calculated from number distribution or the like based on measurement by a laser diffraction/scattering method or the like. To be specific, when a cumulative curve is determined with the total volume of the population of powders as 100%, 50% diameter (D50) which is a particle size at a point where the cumulative curve reaches 50% may be considered to be as the mean particle size.

The biocompatible particle produced with PLGA is easy to migrate to endothelial cells, white blood cells, cardiac muscle cells, inflammatory cells, regions with high vascular permeability, or the like; and is therefore preferred in that the enclosed mitochondrial damage inhibitor and anti-inflammatory agent are sustainably released in endothelial cells, white blood cells, cardiac muscle cells, inflammatory cells, regions with high vascular permeability, or the like.

Further, the above biocompatible particle may be produced by using polyethylene glycol (PEG) modifications of PLGA. When the surface of PLGA is modified with PEG, the stability in the blood is increased. In this respect, the PEG modification is preferred.

The above biocompatible particle can be produced by any method as long as the method is a method capable of processing the biocompatible polymer and at least one of the mitochondrial damage inhibitor and the anti-inflammatory agent into particles with a number mean particle size of less than 1,000 nm, for example, 2.5 to 1,000 nm, preferably 5 to 800 nm, more preferably 25 to 500 nm, still more preferably about 50 to 300 nm, and most preferably 100 to 250 nm, when measured by, for example, a laser diffraction/scattering method or the like. The biocompatible particle can be produced by, for example, spherical crystallization technique. The spherical crystallization technique is a technique of making particles wherein crystals precipitated and deposited during crystallization operation are formed into spherical shape. According to the spherical crystallization technique, the produced compound can be processed while physical properties of the compound are being controlled. Among the spherical crystallization technique, there is, for example, an emulsion solvent diffusion method (hereinafter referred to simply as "ESD method").

In the ESD method, two kinds of solvents are used: a good solvent which dissolves the biocompatible polymer that encloses at least one of mitochondrial damage inhibitor and the anti-inflammatory agent and a poor solvent which does not dissolve the biocompatible polymer. For the good solvent, organic solvents that dissolve the biocompatible polymer and are miscible with the poor solvent are used. The kind of the good solvent and the poor solvent is determined depending on the kind or the like of substances that are enclosed; and the kind of the good solvent and the poor solvent is not particularly restricted. Because the biocompatible particle according to the present embodiment is used as a raw material of compositions that act mainly on the human body, it is preferred that those having a high safety to the human body and exhibiting less environmental burden be used.

Examples of the poor solvent include water and water with an added surfactant. As the surfactant, an aqueous polyvinyl alcohol solution is for example preferred. As surfactants other than polyvinyl alcohol, examples include lecithin, hydroxymethyl cellulose, and hydroxypropyl cellulose. It is to be noted that, when polyvinyl alcohol remains, polyvinyl alcohol may be removed by centrifugation or the like after the evaporation of the solvent.

Examples of the good solvent include halogenated alkanes, acetone, methanol, ethanol, ethyl acetate, diethyl ether, cyclohexane, benzene, and toluene, which are organic solvents with a low boiling point and poor water solubility. Preferably, acetone which exhibits less adverse effects on the environment or the human body is used solely or as a mixed solution with ethanol.

Production of drug-enclosing particles that encloses the mitochondrial damage inhibitor by the ESD method will be described below. In the ESD method, a biocompatible polymer is first dissolved in a good solvent; and in order for the biocompatible polymer not to precipitate, a mitochondrial damage inhibitor solution is thereafter added to and mixed with the good solvent. When the mixed solution containing the biocompatible polymer and the mitochondrial damage inhibitor is, while stirred, added dropwise to a poor solvent, the good solvent in the mixed solution rapidly diffuses and migrates into the poor solvent. As a result, the good solvent is emulsified in the poor solvent to form emulsion droplets of the good solvent with a diameter of about several micrometres. Further, because the organic solvent continuously diffuses from the inside of the emulsion to the poor solvent due to mutual diffusion between the good solvent and the poor solvent, the solubility of the biocompatible polymer and the mitochondrial damage inhibitor in the emulsion droplet decreases. Eventually, biocompatible particles that are spherical crystal particles enclosing the mitochondrial damage inhibitor are generated. Thereafter, the organic solvent, which is the good solvent, is removed by centrifugation or evaporation under reduced pressure to obtain biocompatible particle powder. The obtained powder is used as is or is, as necessary, subjected to composite formation via freeze drying or the like to yield aggregation particles capable of being redispersed. The composite particle is filled in a vessel as the drug-enclosing particle. In the thus obtained drug-enclosing particle, the mitochondrial damage inhibitor is preferably enclosed in 0.1 to 99% (w/v), more preferably 0.1 to 30% (w/v), still more preferably 1 to 10% (w/v), and in particular preferably 2 to 3% (w/v).

Note that the drug particles that enclose an anti-inflammatory agent can be produced in the same manner using an anti-inflammatory agent solution by the ESD method. Further, in the above ESD method, if the biocompatible polymer is dissolved in the good solvent and then the mitochondrial damage inhibitor solution and the anti-inflammatory agent solution are added to and mixed with the good solvent, the drug-enclosing particle that encloses both of the mitochondrial damage inhibitor and the anti-inflammatory agent can be produced.

Because the obtained drug-enclosing particle is substantially spherical in the above spherical crystallization technique, there is no need to consider a problem concerning residual catalysts or raw material compounds. In addition, according to the spherical crystallization technique, it is possible to readily form the drug-enclosing particles with less variation of particle size.

It is to be noted that, for the purpose of increasing the enclosing percentage of the mitochondrial damage inhibitor and at least one of the anti-inflammatory agents inside of the biocompatible particle, cationic polymers may be added to the poor solvent. In cases where the cationic polymer is added in the poor solvent, it is thought that the cationic polymer adsorbed on the surface of the biocompatible particle interacts with the mitochondrial damage inhibitor or the anti-inflammatory agent present on the surface of the emulsion droplet, which allows for prevention or reduction of leakage of the mitochondrial damage inhibitor or the anti-inflammatory agent into the poor solvent.

Examples of the cationic polymer include chitosan and chitosan derivatives; cationized cellulose in which plural cationic groups are bound to cellulose; polyamino compounds such as polyethyleneimine, polyvinylamine, or polyallylamine; polyamino acids such as polyornithine or polylysine; polyvinyl imidazole, polyvinyl pyridinium chloride, alkylamino methacrylate quaternary salt polymer (DAM), and alkylamino methacrylate quaternary salt·acrylamide copolymer (DAA). In particular, chitosan or derivatives thereof are suitably used.

Chitosan is a natural polymer in which a large number of glucosamines are linked, which glucosamine is a type of sugar with an amino group; and has characteristics of emulsion stability, shape retainability, biodegradability, biocompatibility, antimicrobial activities, and the like; and thus widely used as raw materials of cosmetics, food products, clothing, pharmaceutical products, and the like. Addition of this chitosan to the poor solvent enables the production of the drug-enclosing particles that exhibit no adverse effects to organisms and are highly safe.

The drug-enclosing particle obtained as described above can be subjected to composite formation to yield aggregate particles (nanocomposites) capable of being redispersed upon powderization by freeze drying or the like. At that time, it is preferred that an organic or inorganic substance be turned into the composite capable of being redispersed and dried together with the drug-enclosing particle. For example, by using sugar alcohol or sucrose, variation of enclosing percentage can be effectively prevented and, at the same time, the sugar alcohol or the like can serve as an excipient to increase ease of handling of the drug-enclosing particle. Examples of the sugar alcohol include mannitol, trehalose, sorbitol, erythritol, maltitose, and xylitose. Of these, trehalose is in particular preferred.

This composite formation allows drug-enclosing particles to become easy-to-handle aggregation particles. The aggregated drug-enclosing particles, in use, return to the particle form by making contact with water and exhibit their characteristics such as high reactivity. It is to be noted that, in place of a freeze drying method, a fluidized bed dry granulation method using, for example, Agromaster AGM (manufactured by Hosokawa Micron Corporation) can be employed to form the composite to achieve integration in a state where redispersion is feasible.

The pharmaceutical composition according to the present embodiment is produced by a known method and contain, as an active component, the drug-enclosing particles at about 0.1% to 99%, 1% to 50%, and preferably 1 to 20% (% refers to % by weight).

The pharmaceutical composition according to the present embodiment is preferably injections, rectal suppositories, vaginal suppositories, transnasal absorption preparations, transdermal absorption preparations, pulmonary absorption preparations, intraoral absorption preparations, oral administration preparations and the like. In this case, such a composition may be formulated with, for example, a pharmaceutically acceptable carrier to be used as a combination drug product. The pharmaceutically acceptable carrier is various organic carrier substances or inorganic carrier substances that are used as formulation materials. The pharmaceutically acceptable carrier is formulated in therapeutic agents for oxidative stress diseases, for example, as an excipient, a lubricant, a binder, a disintegrant in the case of solid preparations; or as a solvent, a solubilizing agent, a suspending agent, an isotonic agent, a buffering agent, a soothing agent in the case of liquid preparations. In addition, additives such as antiseptics, antioxidants, coloring agents, or sweeteners can be used as necessary.

The excipient is, for example, lactose, white sugar, D-mannitol, starch, crystalline cellulose, light anhydrous silicic acid, or the like. The lubricant is, for example, magnesium stearate, calcium stearate, talc, colloidal silica, or the like. The binder is, for example, crystalline cellulose, white sugar, D-mannitol, dextrin, hydroxypropyl cellulose, hydroxy propyl methylcellulose, polyvinylpyrrolidone, or the like. The disintegrant is, for example, starch, carboxymethyl cellulose, carboxymethyl cellulose calcium, croscarmellose sodium, carboxymethyl cellulose sodium, or the like.

The solvent is, for example, water for injection, alcohol, propylene glycol, macrogol, or the like. The solubilizing agent is, for example, polyethylene glycol, propylene glycol, D-mannitol, benzyl benzoate, ethanol, tris aminomethane, cholesterol, triethanolamine, sodium carbonate, sodium citrate, or the like. The suspending agent is a surfactant, a hydrophilic polymer, or the like, and is, for example, stearyltriethanolamine, sodium lauryl sulfate, laurylamino propionic acid, lecithin, benzalkonium chloride, benzethonium chloride, glyceryl monostearate, polyvinyl alcohol, polyvinylpyrrolidone, sodium carboxymethyl cellulose, methylcellulose, hydroxymethyl cellulose, hydroxyethyl cellulose, hydroxypropyl cellulose, or the like.

The isotonic agent is, for example, sodium chloride, glycerin, D-mannitol, or the like. The buffering agent is, for example, phosphate buffer, acetate buffer, carbonate buffer, citrate buffer, or the like. The soothing agent is, for example, benzyl alcohol, or the like. The antiseptic is, for example, para-hydroxybenzonates, chlorobutanol, benzyl alcohol, phenethyl alcohol, dehydroacetic acid, sorbic acid, or the like. The antioxidant is, for example, sulfite, ascorbic acid, or the like.

It is suitable that the pharmaceutical composition according to the present embodiment is used in, for example, treatment or prevention of disorders caused by ischemia. The disorder caused by ischemia includes, for example, ischemic injury, ischemia-reperfusion injury, and the like. The disorder caused by ischemia also includes abnormal conditions, cell damages, vascular disorders, organ failure, and the like that are caused by lowered oxygen supply to tissues, swelling of cells and intracellular organelles, disruption of mitochondrial membranes and lysosomal membranes, which are ascribed to ischemia; and, in addition, abnormal conditions, cell damages, vascular disorders, and organ failure that are induced by reperfusion. Here, the ischemic site is preferably, the organ such as the heart, the lung, the kidney, or the brain. In this case, the disorder caused by ischemia is a disorder in the organ that has come to be in an ischemic state.

In particular, the pharmaceutical composition according to the present embodiment, as shown in the examples described below, reduces the infarct size (the dimension of infarct) of the organ that has come to be in an ischemic state. Also in this regard, it is suitable that such a pharmaceutical composition is used in the treatment or prevention of disorders caused by ischemia. The infarct size is the most important factor that determines the prognosis of the ischemic disease. Because of this, such a pharmaceutical composition decreases cardiogenic shock, lethal arrhythmia, and heart failure in the prognosis of the ischemic disease.

Biocompatible particles that contain PLGA are, as shown in Examples 2 and 3 described below, selectively delivered to the ischemic region in an efficient fashion. Further, when cyclosporine nanoparticles prepared by enclosing cyclosporine in such biocompatible particles as the mitochondrial damage inhibitor and anti-inflammatory agent nanoparticles prepared by enclosing pitavastatin, irbesartan, or pioglitazone in such biocompatible particles as the anti-inflammatory agent are administered to model mice of ischemia reperfusion after ischemia and before reperfusion, the infarct size is drastically reduced (see Examples 6 to 8 described below).

Besides, the pharmaceutical composition according to the present embodiment may be applied to control of rejection in transplantation of organs such as the kidney, the liver, the heart, the lung, and the pancreas, control of rejection and graft versus host disease in bone marrow transplantation, Behcet's disease, psoriasis vulgaris, pustular psoriasis, erythrodermic psoriasis, psoriatic arthritis, aplastic anemia, pure red cell aplasia, nephrotic syndrome, inflammatory diseases involving inflammatory cells, and the like because it is easily migrate to endothelial cells, white blood cells, cardiac muscle cells, inflammatory cells, and regions with high vascular permeability.

A dose of the pharmaceutical composition according to the present embodiment is determined as appropriate in accordance with subjects' gender, age, body weight, symptoms, or the like. Such a pharmaceutical composition is administered at a therapeutically effective amount in terms of the mitochondrial damage inhibitor and the anti-inflammatory agent. An effective amount refers to an amount of the mitochondrial damage inhibitor and the anti-inflammatory agent that is necessary to achieve desired results and an amount necessary to result in delay in progression, inhibition, prevention, reversal, or cure of conditions subjected to therapy or treatment. The dose of such a pharmaceutical composition is typically 0.01 mg/kg to 1,000 mg/kg, preferably 0.1 mg/kg to 200 mg/kg, more preferably 0.2 mg/kg to 20 mg/kg; and can be administered once a day or dividedly in twice or more times. In the case where such a pharmaceutical composition is dividedly administered, the pharmaceutical composition is preferably administered once to four times per day. Further, such a pharmaceutical composition may be administered at various kinds of dosing frequency such as every day, every other day, once a week, every other week, or once a month. It is preferred that the dosing frequency be readily determined by medical doctors. It is to be noted that an amount out of the above range can also be used as necessary.

The route of administration of the pharmaceutical composition according to the present embodiment is not particularly restricted; and it can be administrated by injection, transnasally, transdermally, via lung, orally, or the like. As the route of administration, intravenous administration or intracoronary administration is in particular preferred.

The pharmaceutical composition according to the present embodiment is administrated to patients in combination with reperfusion therapy. For example, such a pharmaceutical composition may be administered to the patient concurrently with the reperfusion therapy or may be administered to the patient several hours, several dozen minutes, or several minutes before the reperfusion therapy is carried out. Such a pharmaceutical composition may be administered to the patient in the door-to-balloon time. Because such a pharmaceutical composition is selectively and promptly delivered to the ischemic region, it is able to significantly reduce the infarct size even when administered to the patient not before the development of ischemia but after the development of ischemia.

As described in detail above, the pharmaceutical composition according to the present embodiment selectively delivers the mitochondrial damage inhibitor and the anti-inflammatory agent that are enclosed in the biocompatible particle to the ischemic site and releases the mitochondrial damage inhibitor and the anti-inflammatory agent. This allows for concurrent intervention in major factors of pathological conditions of the ischemia-reperfusion injury: mitochondrial damages in an early phase following ischemia and inflammation in a late phase following ischemia and therefore enables the ischemia-reperfusion injury to be prevented or reduced. As a result, the infarct size is able to be reduced as small as possible.

In addition, because the pharmaceutical composition according to the present embodiment is able to achieve sufficient pharmacological effects at a lower concentration as compared to cases where the mitochondrial damage inhibitor and the anti-inflammatory agent are not enclosed in the biocompatible particle, it is able to lower the risk of systemic side effects of the mitochondrial damage inhibitor and the anti-inflammatory agent, for example, kidney toxicity, hypertension, or the like in the case of using cyclosporine and to increase safety.

Further, in the present embodiment, the biocompatible particle may contain PLGA with a number mean particle size of 2.5 to 1,000 nm or a PEG modification thereof as well. PLGA is suitable for medicine applications, in particular, administration to the human body because PLGA exhibits biocompatibility of being less stimulative and less toxic to organisms and biodegradability of being broken down after administration to be metabolized. Further, the PEG modification of PLGA has improved stability in the blood and is therefore useful for optimizing pharmacokinetics profile such as metabolism absorption stability. In addition, because PLGA and the PEG modification of PLGA are delivered to and accumulated in endothelial cells, white blood cells, cardiac muscle cells, inflammatory cells, and regions with high vascular permeability, and allow sustained release of their enclosing drug, the drug enclosed is able to be selectively and safely delivered to a targeted site.

It is to be noted that the selectively mitochondrial damage inhibitor may be cyclosporine in the present embodiment. In the examples described below, cyclosporine is enclosed in the biocompatible particle to be thereby selectively delivered to the ischemic region and exerts an excellent effect of reducing the infarct size.

Further in the present embodiment, the disorder caused by ischemia may be disorders in organs that have come to be in an ischemic state. The biocompatible particle according to the present embodiment, as shown in the examples described below, has high blood vessel permeability and is selectively delivered to an organ ischemic region where inflammatory cells pile up. Because of this, the biocompatible particle according to the present embodiment produces an organ protective effect on disorders in the organ that has come to be in an ischemic state, which organ includes the lung, the kidney, and the brain, in addition to the heart.

It is to be noted that the drugs that are enclosed in the biocompatible particle may be two or more kinds of the mitochondrial damage inhibitors or two or more kinds of the anti-inflammatory agents; and two or more kinds of the mitochondrial damage inhibitors and two or more kinds of the anti-inflammatory agents may be enclosed in combination. In addition, other drugs, for example, antibiotics, analgesics, vitamins, or the like may be enclosed in the biocompatible particle, which drugs excluding drugs that are not desirable to be used in combination with the mitochondrial damage inhibitor and the anti-inflammatory agent.

### (Embodiment 2)

Next, the embodiment 2 of the present disclosure will be described.

The method of treating a disorder caused by ischemia according to the present embodiment comprises the step of administering to a patient with a mitochondrial damage inhibitor enclosed in a biocompatible particle and an anti-inflammatory agent enclosed in a biocompatible particle in combination. As long as the mitochondrial damage inhibitor and the anti-inflammatory agent are each enclosed in the biocompatible particle, they may be administered, as an active component, as a pharmaceutical composition that contains a pharmaceutically acceptable carrier or the like.

The phrase "in combination" means that, for example, the mitochondrial damage inhibitor enclosed in the biocompatible particle and the anti-inflammatory agent enclosed in the biocompatible particle are administered at the same time; such a mitochondrial damage inhibitor and such an anti-inflammatory agent are successively administered at several-second or several-minute intervals; or such a mitochondrial damage inhibitor and such an anti-inflammatory agent are formulated into and administered as a combination drug product.

The route of administration of the above mitochondrial damage inhibitor and anti-inflammatory agent is not particularly restricted; and it can be administrated by injection, transnasally, transdermally, via lung, orally, or the like. Intravenous administration or intracoronary administration is in particular preferred as the route of administration of the above mitochondrial damage inhibitor and anti-inflammatory agent.

The method of treating a disorder caused by ischemia may comprise the step of carrying out reperfusion therapy. In that case, the above administration step is preferably carried out concurrently with the reperfusion therapy or several hours, several dozen minutes, or several minutes before the reperfusion therapy is carried out.

As described in detail above, because the mitochondrial damage inhibitor and the anti-inflammatory agent that are enclosed in the biocompatible particle is selectively delivered to the ischemic site, the treatment method according to the present embodiment is able to intervene at the same time in the mitochondrial damage in an early phase following ischemia and the inflammation in a late phase after ischemia, which are major factors of pathological conditions of the ischemia-reperfusion injury. This intervention makes it possible to attain the maximum effect of reducing the infarct size based on the mitochondrial damage inhibitor and the anti-inflammatory agent.

### EXAMPLES

By way of the following examples, the present disclosure will be more specifically described; but the present disclosure is not limited by the examples.

### (Example 1: Preparation of drug-enclosing nanoparticles)

In a mixed solvent of 40 ml of acetone and 20 ml of ethanol, 1.2 g of PLGA (manufactured by Wako Pure Chemical Industries, Ltd., PLGA7520, lactic acid: glycolic acid = 75:25, weight average molecular weight 20,000) and 0.05 g of cyclosporine A (manufactured by Sigma, hereinafter also referred to simply as "CsA") were dissolved to obtain a polymer solution. This solution was added dropwise, at a constant rate (4 ml/min), to 120 ml of 0.5 wt% PVA solution that was stirred at 40°C and 400 rpm, thereby obtaining a cyclosporine nanoparticle (CsA-NP) suspension. Subsequently, while the suspension was continuously stirred at 40°C and 100 rpm under reduced pressure, the mixed solvent was evaporated to be removed. After the mixed solvent was evaporated and removed for about two hours, the suspension was subjected to filter filtration (opening 32 µm); and the filtrate was subjected to freeze drying overnight to obtain dried powder of CsA-NP. The obtained dried powder had a number mean particle size of 221 nm and exhibited an enclosing percentage of CsA of 2.67% (w/v) based on PLGA.

A pitavastatin nanoparticle (pitavastatin-NP) was prepared by using 46.2 g of PLGA and 10.1414 g of pitavastatin (manufactured by Kowa Company, Limited) in the same manner as described for the above CsA-NP. The obtained dried powder of pitavastatin-NP had a number mean particle size of 180 nm and exhibited an enclosing percentage of pitavastatin of 12% (w/v) based on PLGA.

An irbesartan nanoparticle (irbesartan-NP) was prepared by using 2 g of PLGA and 150 mg of irbesartan (manufactured by Shionogi & Co., Ltd.) in the same manner as described for the above CsA-NP. The obtained dried powder of irbesartan-NP had a number mean particle size of 234 nm and exhibited an enclosing percentage of irbesartan of 3.29% (w/v) based on PLGA.

A pioglitazone nanoparticle (pioglitazone-NP) was prepared by using 2 g of PLGA and 100 mg of pioglitazone (manufactured by Takeda Pharmaceutical Company Limited) in the same manner as described for the above CsA-NP. The obtained dried powder of pioglitazone-NP had a number mean particle size of 380 nm and exhibited an enclosing percentage of pioglitazone of 3.7% (w/v) based on PLGA.

CCR2 inhibitor-NP was prepared by using 20 to 100 g of PLGA and 5 to 20 g of BMS CCR2 22(2- [(Isopropylaminocarbonyl)amino]-N-[2-[[cis-2-[[4-(methylthio)benzoyl]amino]cycl ohexyl]amino]-2-oxoethyl] -5-(trifluoromethyl)benzamide, manufactured by Bristol-Myers Squibb) in the same manner as described for the above CsA-NP. The obtained dried powder of CCR2 inhibitor-NP had a number mean particle size of 179 nm and exhibited an enclosing percentage of CCR2 inhibitor of 3.33% (w/v) based on PLGA.

FITC nanoparticle (FITC-NP) which enclosed a fluorescent marker (FITC) was produced in the same manner as described for the above CsA-NP. The obtained dried powder of FITC-NP had a number mean particle size of 225 nm and exhibited an enclosing percentage of FITC of 5% (w/v) based on PLGA.

In this example, the definition of the number mean particle size was defined as follows. A cumulative curve was determined with the total volume of the population of the powder as 100%, 50% diameter (D50) was defined as a parameter for generally evaluating particle size distribution as a cumulative median diameter (mean particle size), which 50% diameter was a particle size at a point where the cumulative curve reached 50%. The mean particle size was measured by subjecting a sample prepared by suspending particles in distilled water to a light scattering method using Microtrack UPA150 (manufactured by Nikkiso Co., Ltd.).

### (Example 2: Verification of selective drug delivery to ischemia reperfusion sites)

Mice (C57BL/6J, 8 to 10 weeks of age, male, 25 to 30 g, n = 3) were intraperitoneally administered with pentobarbital sodium (60 mg/kg) or subjected to inhalation anesthesia with isoflurane. During the procedure, respiration was controlled by a mechanical ventilator (tidal volume: 0.5 ml, respiratory rate: 140 breaths/min); the mouse was kept warm using a heating pad so that the rectal temperature was maintained at 36.8°C to 37.2°C. An incision was made horizontally at the level of the third to fourth intercostal space; and the pericardium was opened. Into a silicone tube with an outside diameter of 1 mm, 8-0 silk suture was passed to form a snare shape; and ligature of left anterior descending coronary artery was performed at the inferior margin of the left atrial appendage. ST change in electrocardiogram and a change in the color of cardiac muscles were observed and ischemia in the cardiac muscles was checked. The silicone tube was removed 30 minutes after the ligature. Reperfusion was carried out and at the same time 0.1 to 0.2 ml of solution of test substance was intravenously injected. The chest was closed using 5-0 silk suture; and then extubation was performed after spontaneous breathing was confirmed. The intravenously-injected test substance is FITC (40 mg/kg) or FITC-NP (800 mg/kg). Note that, as a control, normal saline was injected to the mouse.

Twenty-four hours after the reperfusion, the mouse was anesthetized again and endotracheal intubation was conducted, followed by thoracotomy. The ligature of the left anterior descending coronary artery was performed at the same site in the same manner as described above; and 2% Evans blue was administered from the inferior vena cava for the purpose of making the ischemic region (Area at Risk; AAR) clear. Subsequently, the heart was promptly taken out and frozen at -80°C. The left ventricle was divided into pieces with a thickness of 1 mm in the short axis direction and five sections were cut out as specimens. Cardiac muscles in the obtained specimen were stained in a 1% triphenyl tetrazolium chloride (TTC) solution (37°C, for 10 minutes). In addition, the specimen of the group administrated with FITC-NP was treated with a reagent for staining the nucleus, DAPI (Vectashield, H1200). In evaluation of the specimen, SMZ1500 (manufactured by Nikon Corporation) was employed as an optical and fluorescence microscope.

### (Results)

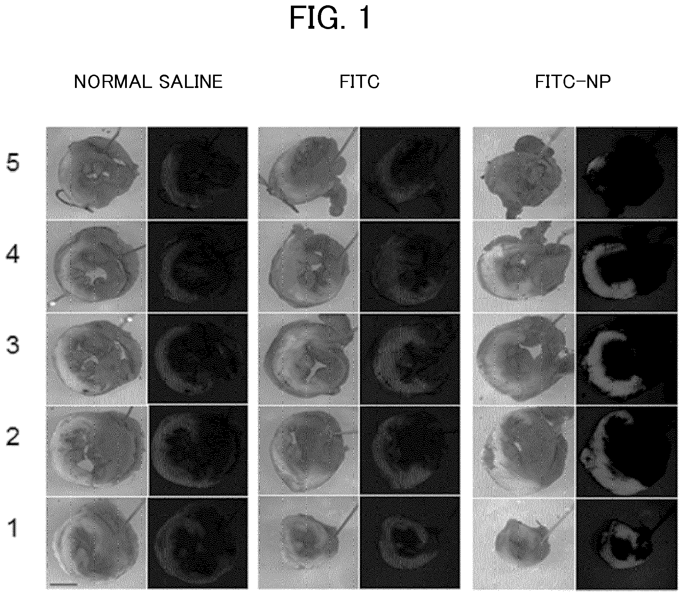

FIG. 1 shows the specimen observed under a stereo microscope. In the specimen of the group administrated with FITC, the fluorescence of FITC was not observed in the infarction site (white) where Evans blue/TCC staining was confirmed. On the other hand, in the specimen of the group administrated with FITC-NP, the fluorescence of FITC was observed consistently with the infarction site (white).

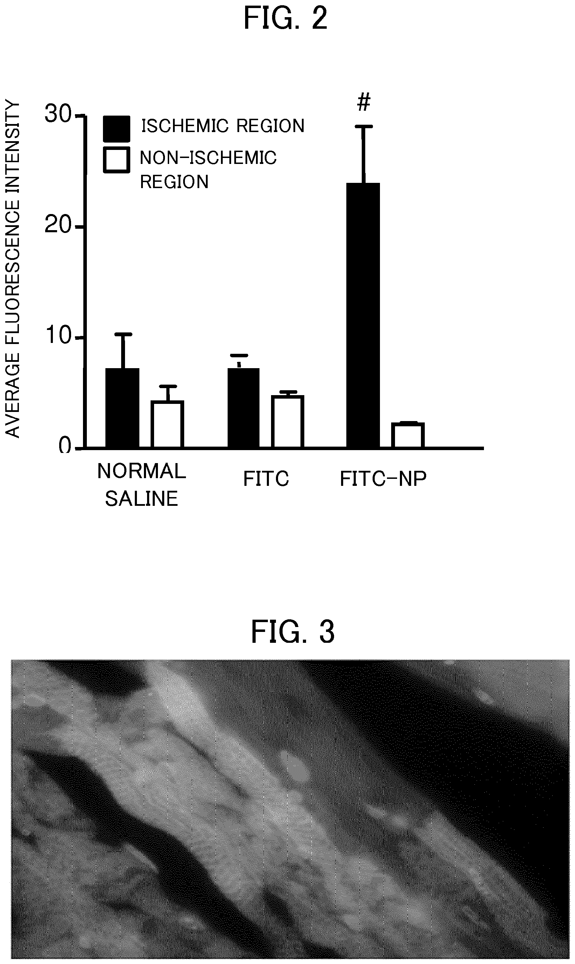

FIG. 2 shows the fluorescence intensity acquired by image analysis for the above specimen. A strong FITC signal was obtained only in the ischemic site of the group administrated with FITC-NP.

FIG. 3 shows a fluorescence image of the specimen of the group administrated with FITC-NP. FITC-NP was shown to migrate into the inside of cardiac muscle cells. From the above results, it has demonstrated that the PLGA nanoparticle is selectively delivered to cardiac muscle cells in the ischemia reperfusion region.

### (Example 3: Distribution of FITC-NP in cardiac muscles in model mice of myocardial ischemia-reperfusion)

In order to study the subcellular distribution of FITC-NP, the above model mouse of myocardial ischemia-reperfusion was, concurrently with the reperfusion, injected intravenously with FITC (0.06 mg in 5.0 ml/kg normal saline) or FITC-NP (1.4 mg of PLGA containing FITC at 0.06 mg/kg in 5.0 ml/kg normal saline); and mitochondria were isolated from the ischemic cardiac muscle five minutes after the reperfusion. Mitochondria were isolated by using Mitochondria Isolation Kit for Tissue (ab110168, Abcam, Massachusetts, the United States) in accordance with a protocol of the kit. The harvested heart was, while cooled with ice, cut into fine pieces, immersed in a solution prepared by adding EDTA (1 mM) to 1 ml of homogenization buffer (10 mM tris, 250 mM sucrose, protease inhibitor, pH7.5), and homogenized using a Dounce homogenizer. The homogenate was centrifuged at 1000 X g at 4°C for five minutes; and the obtained supernatant was further centrifuged at 1500 X g for 15 minutes to obtain the pellet (mitochondrial fraction). The obtained pellet was washed twice with the homogenization buffer.

### (Results)

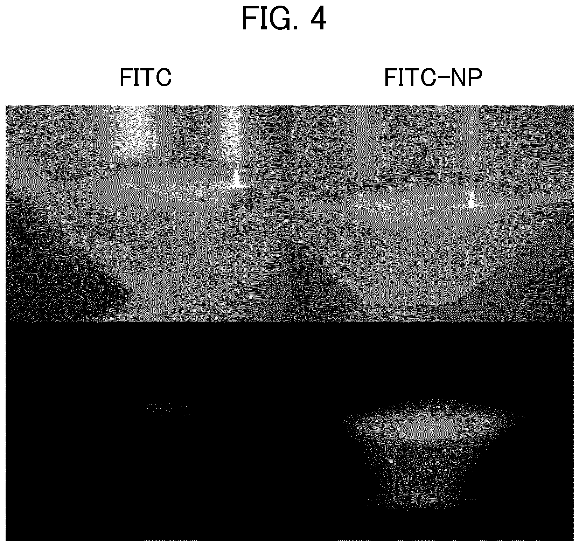

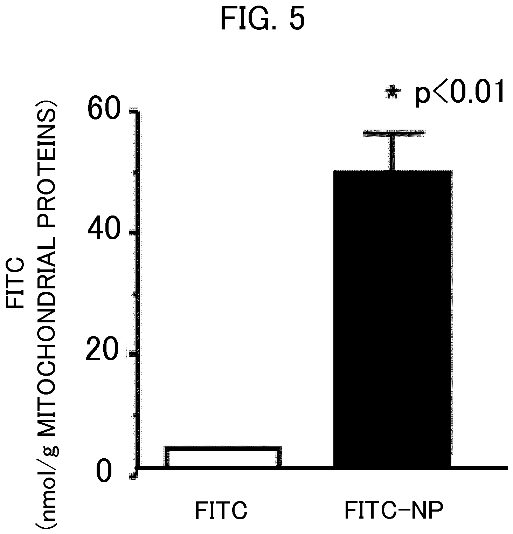

FIG. 4 shows an optical image and a fluorescence image of the mitochondrial fraction. More fluorescence was found in the mitochondrial fraction in FITC-NP as compared to that in unenclosed FITC. FIG. 5 shows the amount of FITC (in mole) based on the weight of proteins in the mitochondrial fraction. As for the amount of FITC based on the amount of mitochondria, FITC-NP exhibited a significantly higher amount than the unenclosed FITC.

### (Example 4: Uptake of FITC-NP by primary cultured cardiac muscle cells)

In order to examine cellular uptake of FITC-NP and subcellular localization of FITC-NP, a primary culture of rat cardiac muscle cells was employed. Ventricular muscle cells of a neonatal rat were prepared from the ventricle of a neonatal Sprague Dawley rat by reference to a method of Fujino *et al.* (Fujino T et al., Recombinant mitochondrial transcription factor A protein inhibits nuclear factor of activated T cells signaling and attenuates pathological hypertrophy of cardiac myocytes., Mitochondrion, 2012, 12, 449-458).

The neonatal rat was euthanized under isoflurane anesthesia; and the heart was quickly taken out and broken down. Tissue were broken down with trypsin (manufactured by Wako Pure Chemical Industries, Ltd.) and collagenase type 2 (manufactured by Worthington, New Jersey, the United States); and cells were suspended in Dulbecco's Modified Eagle's Medium (hereinafter referred to as "DMEM medium", manufactured by Sigma -Aldrich) containing 10% fetal bovine serum (hereinafter referred to simply as "FBS", manufactured by Thermo Scientific, Massachusetts, the United States), penicillin (manufactured by Invitrogen, California, the United States), and streptomycin (manufactured by Invitrogen). The suspended cells were plated in a 100 mm culture dish (Cellstar (trademark), manufactured by Greiner Bio-One, North Carolina, the United States) twice and left to stand each time for 70 minutes to decrease the number of cells that were not cardiac muscle cells. Nonadherent cardiac muscle cells were plated in a culture dish (Primaria (trademark), Falcon) so as to have an optimal density for each experiment and maintained at 37°C in an environment of humidified air containing 5% CO₂ for 36 hours.

Subsequently, the cardiac muscle cells were washed with HBSS and treated with 250 nM MitoTracker (trademark) Orange (manufactured by Invitrogen) in the culture medium for 30 minutes. Next, the culture medium was replaced; and the cardiac muscle cells were exposed to 100 µM hydrogen peroxide for 30 minutes in order to mimic an ischemia reperfusion state. The culture dish was washed; and a fresh medium containing FITC-NP (59.0 µg/ml PLGA containing 2.53 µg/ml FITC) was added to the culture dish. After 30 minutes, the plate was washed with PBS and fixed with methanol at -20°C for 20 minutes. The resultant was then labeled with a medium that contains DAPI (Vectashield (trademark), manufactured by Vector Laboratories) and observed by using a confocal microscope (A1, manufactured by Nikon Corporation).

DAPI was detected at 405 nm; MitoTracker (trademark) Orange was at 561 nm; and FITC was at 457 nm. The fluorescence emitted light was collected using two photomultiplier tube adjusted to 425 to 475 nm (DAPI), 565 to 615 nm (MitoTracker (trademark) Orange), or 500 to 530 nm (FITC) by a band-pass filter. The observation was all carried out using a 60x oil-immersion objective lens. Multicolor images were obtained by using each excitation wavelength and switching fluorescence channels.

### (Results)

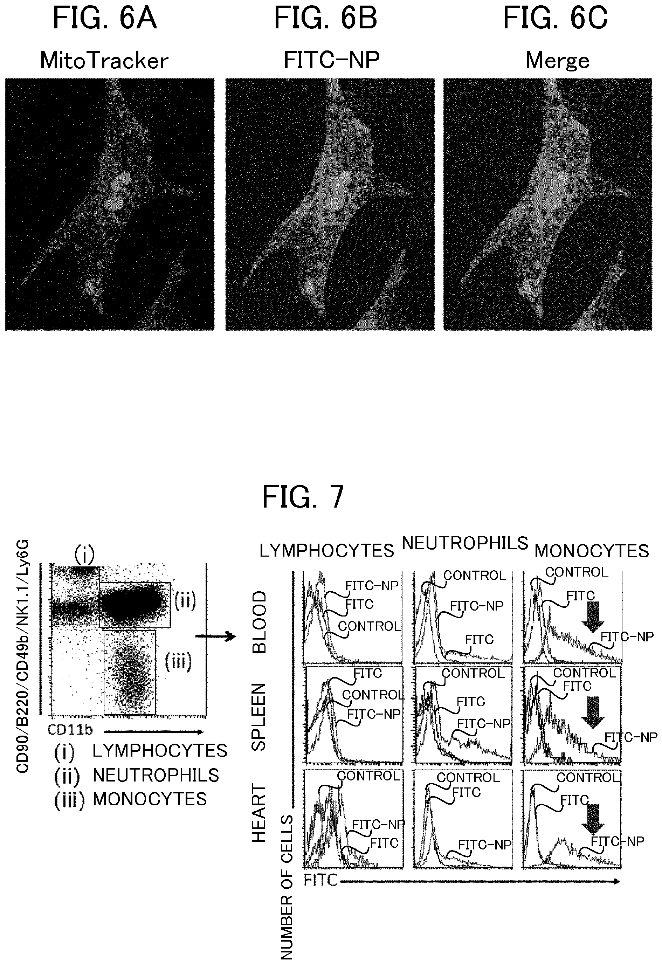

As shown in FIG. 6A, FIG. 6B and FIG. 6C, a large amount of FITC-NPs were taken up in the cardiac muscle cells that had been treated with 100 µM hydrogen peroxide (100 cardiac muscle cells were evaluated for each group). In addition, the fluorescence of FITC was localized at mitochondria.

In this example, the exposure to hydrogen peroxide which mimicked oxidative stress in ischemia reperfusion also promoted the uptake of PLGA nanoparticles into the mitochondria of rat cardiac muscle cells in vitro. The ischemia reperfusion in cardiac muscles is reportedly attributed to the stationary character and loss of mitochondrial membrane potential. By this, it is thought that anionic PLGA particles that are delivered to cardiac muscle cells and mitochondria of the ischemia reperfusion relatively increase.

### (Example 5: Flow cytometry analysis using FITC-NP)

In accordance with the above Example 2, mice were administered with normal saline, FITC, or FITC-NP, subjected to reperfusion, and put down by euthanasia 24 hours later (n= 3 for each group). White blood cells (monocytes, neutrophils, and lymphocytes) in the blood, the spleen, and the heart were analyzed by flow cytometry. The monocyte was defined as CD11bhi (CD90/B220/CD49b/NK1.1/Ly-6G)1oLy-6Chi/lo; the neutrophil was as CD 11 bhi (CD90/B220/CD49b/NK1.1/Ly-6G)hiLy-6Cint; and the lymphocyte was as CD11blo (CD90/B220/CD49b/NK1.1/Ly-6G)hi. FACSCalibur (manufactured by BD Bioscience) was employed for the measurement; and Cell Quest software (manufactured by BD Bioscience) was for the analysis.

### (Results)

FIG. 7 shows the FITC signal detected by the flow cytometry analysis for the white blood cell in the blood, the spleen, and the heart. In the monocyte, a stronger FITC signal was found in the group administered with FITCNP as compared to in the group administered with FITC.

By this example, it has been shown that the PLGA particle is also selectively delivered to activated monocytes that are recruited to the ischemia reperfusion site.

### (Example 6: Drug efficacy evaluation of concomitant administration of CsA-NP and pitavastatin-NP)

In the same manner as described in Example 2, ischemia was produced in the cardiac muscle in mice (n= 8) and reperfusion was carried out. A solution of test substance was intravenously administered 0 to 5 minutes before reperfusion. As for the test substance that was intravenously injected, in the case of single agent administration, CsA (1.0 mg/kg), pitavastatin (0.3 mg/kg), CsA-NP (1.0 mg/kg), and pitavastatin-NP (0.3 mg/kg) were used; and in the case of combined use of two agents, CsA (1.0 mg/kg) and pitavastatin (0.3 mg/kg), CsA-NP (1.0 mg/kg) and pitavastatin (0.3 mg/kg), and CsA-NP (1.0 mg/kg) and pitavastatin-NP (0.3 mg/kg) were employed. Note that normal saline was, as a control, administered to the mouse.

Each of the sections was stained in the same manner as described above; and the areas of infarct (region shown in white) and AAR (region shown in red) were each measured and the area of the infarct region/the area of AAR was calculated.

### (Results)

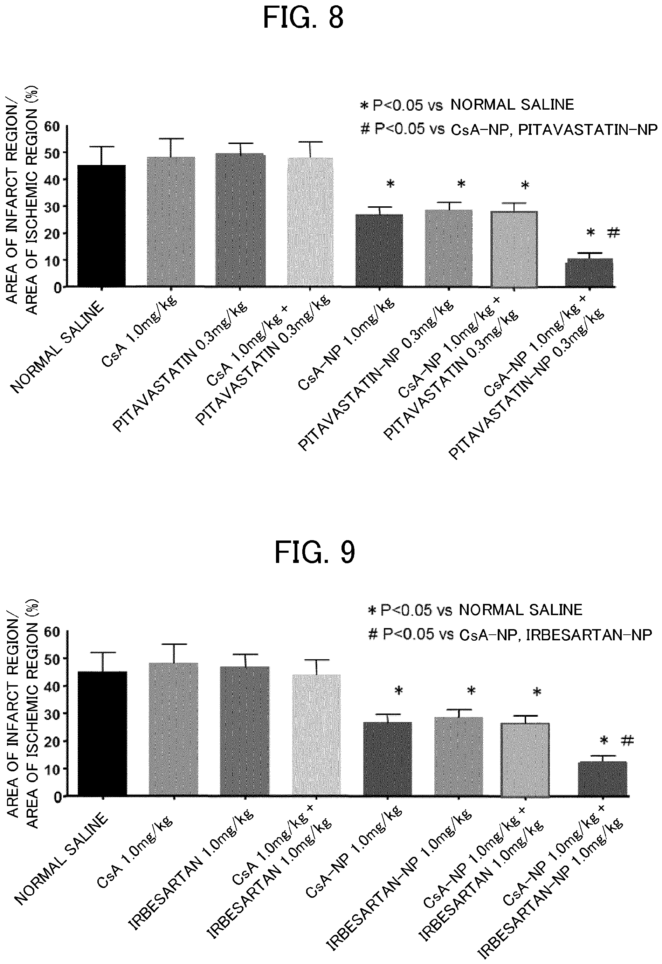

As shown in FIG. 8, when CsA-NP and pitavastatin-NP were used in combination, a drastic effect of reducing the infarct size was found. This is because the effective amount of the mitochondrial damage inhibitor and the anti-inflammatory agent are delivered to the reperfusion region by the PLGA particle. The administration of the mitochondrial damage inhibitor and the anti-inflammatory agent is able to intervene at the same time in mitochondrial damage in an early phase following ischemia and inflammation in a late phase after ischemia, which are major factors of pathological conditions of the ischemia-reperfusion injury; and therefore the ischemia-reperfusion injury was sufficiently prevented or reduced and the infarct size was thus drastically reduced.

Note that, in each case of the single agent administration of pitavastatin, the single agent administration of pitavastatin-NP, the combined use of two agents CsA and pitavastatin, and the combined use of two agents CsA-NP and pitavastatin, even when pitavastatin was dosed at the maximum soluble amount of 10 mg/kg, the drastic reduction of the infarct size was not seen. In addition, when the dose of pitavastatin was 0.1 mg/kg, significant reduction of the infarct size was not seen in each case of the single agent administration of pitavastatin-NP and the combined use of two agents CsA-NP and pitavastatin-NP.

### (Example 7: Drug efficacy evaluation of concomitant administration of CsA-NP and irbesartan-NP)

In the same manner as described in Example 2, ischemia was produced in the cardiac muscle in mice (n= 8) and reperfusion was carried out. A solution of test substance was intravenously administered 0 to 5 minutes before reperfusion. As for the test substance that was intravenously injected, in the case of single agent administration, CsA (1.0 mg/kg), irbesartan (1.0 mg/kg), CsA-NP (1.0 mg/kg), and irbesartan-NP (1.0 mg/kg) were used; and in the case of combined use of two agents, CsA (1.0 mg/kg) and irbesartan (1.0 mg/kg), CsA-NP (1.0 mg/kg) and irbesartan (1.0 mg/kg), and CsA-NP (1.0 mg/kg) and irbesartan-NP (1.0 mg/kg) were employed. Note that normal saline was, as a control, administered to the mouse.

Each of the sections was stained in the same manner as described above; and the areas of infarct and AAR were each measured and the area of the infarct region/the area of AAR was calculated.

### (Results)

As shown in FIG. 9, when CsA-NP and irbesartan-NP were used in combination, a drastic effect of reducing the infarct size was found.

Note that, in each case of the single agent administration of irbesartan, the single agent administration of irbesartan-NP, the combined use of two agents CsA and irbesartan, and the combined use of two agents CsA-NP and irbesartan, even when irbesartan was dosed at the maximum soluble amount of 3.0 mg/kg, the drastic reduction of the infarct size was not seen.

### (Example 8: Drug efficacy evaluation of concomitant administration of CsA-NP and pioglitazone-NP)

In the same manner as described in Example 2, ischemia was produced in the cardiac muscle in mice (n= 3) and reperfusion was carried out. A solution of test substance was intravenously administered 0 to 5 minutes before reperfusion. As for the test substance that was intravenously injected, in the case of single agent administration, CsA (1.0 mg/kg), pioglitazone (1.0 mg/kg), CsA-NP (1.0 mg/kg), pioglitazone-NP (1.0 mg/kg) were used; and in the case of combined use of two agents, CsA (1.0 mg/kg) and pioglitazone (1.0 mg/kg), CsA-NP (1.0 mg/kg) and pioglitazone (1.0 mg/kg), CsA-NP (1.0 mg/kg) and pioglitazone-NP (1.0 mg/kg) were employed. Note that normal saline was, as a control, administered to the mouse.

Each of the sections was stained in the same manner as described above; and the areas of infarct and AAR were each measured and the area of the infarct region/the area of AAR was calculated.

### (Results)

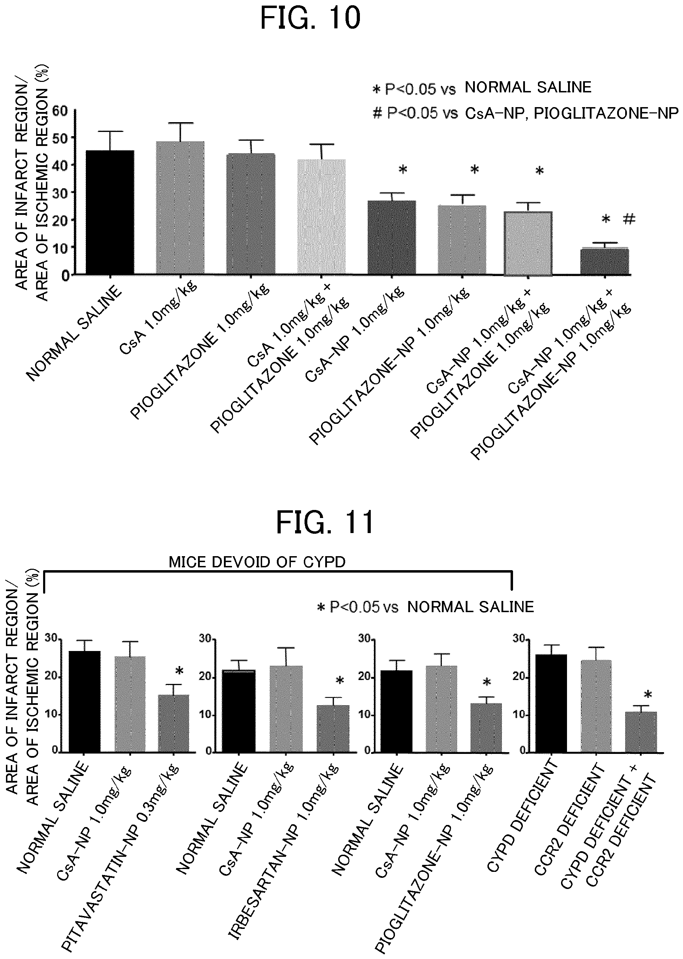

As shown in FIG. 10, when CsA-NP and pioglitazone-NP were used in combination, a drastic effect of reducing the infarct size was found.

Note that, in each case of the single agent administration of pioglitazone-NP, the combined use of two agents CsA and pioglitazone, and the combined use of two agents CsA-NP and pioglitazone, even when pioglitazone was dosed at the maximum soluble amount of 3.0 mg/kg, the drastic reduction of the infarct size was not seen.

### (Example 9: Drug efficacy evaluation using knock-out mice)

Using mice devoid of a major regulatory factor for mPTP, cyclophilin D (CypD), therapeutic effects of the inhibitor of activated monocytes were evaluated. The mice devoid of CypD were obtained from Jackson Laboratories. In the same manner as described in Example 2, ischemia was produced in the cardiac muscle in mice and reperfusion was then carried out 30 minutes later. A solution of a test substance was intravenously injected from five minutes before the reperfusion to the moment of the reperfusion. CsA-NP (1.0 mg/kg), pitavastatin-NP (0.3 mg/kg), irbesartan-NP (1.0 mg/kg), and pioglitazone-NP (1.0 mg/kg) were used as the test substance. Twenty four hours after the reperfusion, the areas of infarct and AAR were each measured and the area of the infarct/the area of AAR was calculated (n= 8). Note that normal saline was, as a control, administered to the mouse.

Further, in addition to mice devoid of CCR2 which is involved in monocyte activation, mice devoid of both CypD and CCR2 were used to evaluate for the infarct size. The mouse devoid of CCR2 and the mouse devoid of both CypD and CCR2 were each produced by breeding.

In the same manner as described above, ischemia was produced in the cardiac muscle in the mouse; and reperfusion was then carried out 30 minutes later. Twenty four hours after the reperfusion, the areas of infarct and AAR were each measured and the area of the infarct region/the area of AAR was calculated (n= 8).

### (Results)

The mouse is devoid of CypD which controls mPTP and thus mPTP does not open. As shown in FIG. 11, even when CsA-NP was administered to the mouse devoid of CypD, no reduction of the infarct size was found. On the other hand, when pitavastatin-NP, irbesartan-NP, or pioglitazone-NP was administered to the mouse devoid of CypD, the infarct size was significantly reduced. This suggests that pitavastatin-NP, irbesartan-NP, and pioglitazone-NP reduce the infarct size via mechanisms that are independent of the inhibition of mitochondrial damage.

In addition, the infarct size of the mouse devoid of both CypD and CCR2 (CypD deficient + CCR2 deficient) was significantly smaller, as compared to that of the mouse devoid of CypD (CypD deficient) or that of the mouse devoid of CCR2 (CCR2 deficient). This result supports the result that "mitochondrial damage inhibition + anti-inflammation drastically reduce the ischemia-reperfusion injury", the result being obtained in the above Examples 6 to 8.

### (Example 10: Drug efficacy evaluation of CCR2 inhibitor-NP)

In the same manner as described in Example 2, ischemia was produced in the cardiac muscle in mice (n= 5 to 8) and reperfusion was carried out. A solution of test substance was intravenously administered 0 to 5 minutes prior to the reperfusion. As for the test substance that was intravenously injected, a CCR2 inhibitor (1.0 mg/kg) and a CCR2 inhibitor-NP (1.0 mg/kg) were used. Note that normal saline was, as a control, administered to the mouse. Further, the maximum soluble amount of bulk of BMS CCR2 22 as a CCR2 inhibitor was 1.0 mg/kg.

Each of the sections was stained in the same manner described above; and the area of infarct and AAR were each measured and the area of infarct region/the area of AAR was calculated.

### (Results)

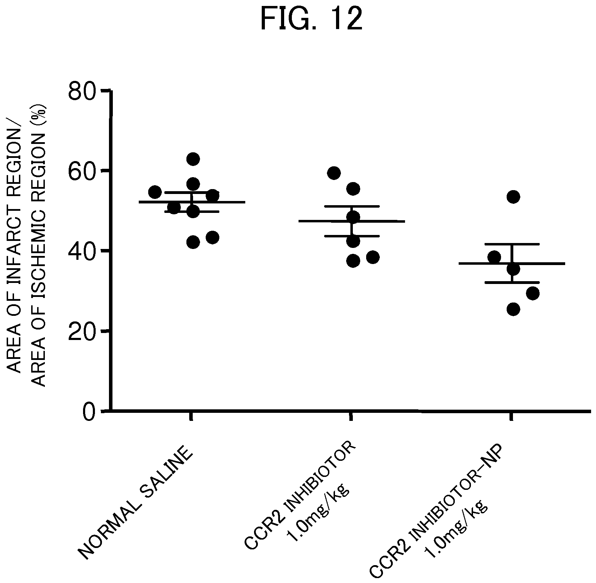

As shown in FIG. 12, the CCR2 inhibitor-NP reduced the infarct size. The above Example 9 shows that CCR2 is a target in the prevention or reduction of the ischemia-reperfusion injury. In this example, by using the CCR2 inhibitor that was enclosed in the nanoparticle it was able to be further confirmed that the inhibition of CCR2 is effective as a drug target in the prevention or reduction of the ischemia-reperfusion injury.

The foregoing describes some example embodiments for explanatory purposes. Although the foregoing discussion has presented specific embodiments, persons skilled in the art will recognize that changes may be made in form and detail without departing from the broader spirit and scope of the invention. Accordingly, the specification and drawings are to be regarded in an illustrative rather than a restrictive sense. This detailed description, therefore, is not to be taken in a limiting sense, and the scope of the invention is defined only by the included claims, along with the full range of equivalents to which such claims are entitled.

The present application is based on Japanese Patent Application No. 2014-139995 filed on July 7, 2014. The specifications, claims, and drawings of Japanese Patent Application No. 2014-139995 are incorporated in the present specification by reference in their entirety.

### Industrial Applicability

The present disclosure is suitable for the treatment of disorders caused by ischemia. By applying the present disclosure, treatment outcome of the disorder caused by ischemia improves and the quality of life of the patient improves.

## Claims

1. A pharmaceutical composition for use in treatment or prevention of a disorder caused by ischemia, the pharmaceutical composition comprising:
a mitochondrial damage inhibitor;
an anti-inflammatory agent; and
a biocompatible particle that encloses both or each of the mitochondrial damage inhibitor and the anti-inflammatory agent.

2. The pharmaceutical composition according to claim 1, wherein
the biocompatible particle comprises
a poly(lactic-co-glycolic acid) copolymer having a number mean particle size of 2.5 to 1000 nm or a polyethylene glycol modification thereof.

3. The pharmaceutical composition according to claim 1 or 2, wherein
the disorder caused by ischemia is
ischemia-reperfusion injury.

4. The pharmaceutical composition according to any one of claims 1 to 3, wherein
the mitochondrial damage inhibitor is
cyclosporine or Mitochondrial division inhibitor 1.

5. The pharmaceutical composition according to any one of claims 1 to 4, wherein
the anti-inflammatory agent is
selected from the group consisting of pitavastatin, irbesartan, pioglitazone, and C-C chemokine receptor type 2 inhibitors.

6. The pharmaceutical composition according to any one of claims 1 to 5, that is administered to a patient in combination with reperfusion therapy.

7. The pharmaceutical composition according to any one of claims 1 to 6, wherein
the disorder caused by ischemia is
a disorder in an organ that has come to be in an ischemic state.
